(19) Europäisches Patentamt
European Patent Office
Office européen des brevets

(11) Veröffentlichungsnummer: **0 090 185**
B1

(12) # EUROPÄISCHE PATENTSCHRIFT

(45) Veröffentlichungstag der Patentschrift: **22.04.87**

(21) Anmeldenummer: **83101909.6**

(22) Anmeldetag: **26.02.83**

(51) Int. Cl.[4]: **C 07 C 121/34,** C 07 D 317/30, C 07 C 69/708, C 07 C 69/734, C 07 C 67/31

(54) **Verfahren zur Herstellung von Acetalen und Enoläthern aus Acryloxymethylenverbindungen.**

(30) Priorität: **30.03.82 DE 3211679**

(43) Veröffentlichungstag der Anmeldung: **05.10.83 Patentblatt 83/40**

(45) Bekanntmachung des Hinweises auf die Patenterteilung: **22.04.87 Patentblatt 87/17**

(84) Benannte Vertragsstaaten: **BE CH DE FR GB IT LI NL**

(56) Entgegenhaltungen:
**EP - A - 0 001 740**
**EP - A - 0 018 473**
**DE - A - 1 793 767**

**HOUBEN-WEYL "Methoden der organischen Chemie, 4. Auflage Band VI, Nr. 3; 1965, GEORG THIEME VERLAG, Stuttgart, Seiten 107,231,232**
**"BULLETIN DE LA SOCIETE CHIMIQUE DE FRANCE", Nr. 11, 1972; J.M. KERN et al. "Etude des aldéhydes al pha-cyanés. al, aldéhyde alpha-cyané non-énolisable. Etude de l'hydratation du groupement carbonyle", Seiten 4379-4383**

(73) Patentinhaber: **DYNAMIT NOBEL AKTIENGESELLSCHAFT, Postfach 1209, D-5210 Troisdorf, Bez. Köln (DE)**

(72) Erfinder: **Peeters, Hermann, Dr., Porzer Strasse 1, D-5216 Niederkassel 3 (DE)**
Erfinder: **Vogt, Wilhelm, Dr., Kleiststrasse 39, D-5000 Köln 40 (DE)**
Erfinder: **Theis, Christoph, Dr., Hoher Rhein 5, D-5216 Niederkassel-Rheidt. (DE)**

EP 0 090 185 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Enoläthern und/oder Acetalen.

Die Herstellung von Enoläthern der Struktur der Formel I ist bekannt, jedoch weisen diese Verfahren Mängel auf. So können nach der europäischen Patentanmeldung 0 018 473 beispielsweise 3-Ethoxyacrylnitril bzw. 3,3-Diethoxypropionitril nur zusammen mit dem unerwünschten und schwer abtrennbaren 3-Ethoxy-2-ethylacrylnitril bzw. 2-(Diethoxymethyl)-butyronitril erhalten werden. Andere Verfahren gehen von teuren Ausgangsstoffen aus oder sind verfahrenstechnisch aufwendig.

Es bestand daher die Aufgabe, aus gut zugänglichen Ausgangsstoffen Enoläther und/oder Acetale in glatt verlaufender Umsetzung als möglichst reine Reaktionsprodukte zu erhalten.

Gegenstand der Erfindung ist ein Verfahren nach Anspruch 1.

Die Acyloxymethylenverbindungen III sind als Ausgangsstoffe durch Acylierung der Hydroxymethylen-Derivate von Nitrilen bzw. ihrer Alkali- oder Erdalkalisalze nach der genannten europäischen Patentanmeldung bzw. durch entsprechende Acylierung von Hydroxymethylenderivaten der Essigsäurester, aliphatischen Carbonsäureester, α-Cyanessigsäureestern, Malonitrilen oder Malonsäureestern bzw. ihrer Alkali- oder Erdalkalisalze, gut zugänglich. Der Rest $R^7$ ist im Falle von $p=1$ der von der Acylgruppe freie Rest einer aliphatischen cycloaliphatischen oder heterocyclischen, ggf. ungesättigten Monocarbonsäure oder, im Falle von $p=2$ der von beiden Acylgruppen freie Rest einer gesättigten oder ggf. ungesättigten, aliphatischen, cycloaliphatischen oder heterocyclischen Dicarbonsäure. Bevorzugte Acylgruppen sind die Acetyl- und die Benzoylgruppe. Als Reste $R^1$ sind niedere Alkylgruppen mit 1 bis 6 C-Atomen und der Rest des Methylglykols, als Reste $R^2$ der des Ethylenglykols bevorzugt. Weitere Reste $R^1$ oder $R^2$ sind dann bevorzugt, wenn der jeweilige Rest im Produkt benötigt wird. Soweit cyclische Ringsysteme Cyc in Frage kommen, sind einkernige bevorzugt und in zweiter Linie bicyclische.

In den mehrkernigen Ringsystemen können die Ringe direkt über ein oder mehrere Atome miteinander oder über ein oder mehrere Kohlenstoff- oder Heteroatome als Brücke miteinander verbunden sein.

Die heterocyclischen Reste enthalten bevorzugt Stickstoff, gegebenenfalls auch Sauerstoff oder weitere Heteroatome. Die gegebenenfalls vorhandenen Substituenten der Ringsysteme sind an sich beliebig, soweit diese bei der Reaktion inert sind. Bevorzugt sind niedere Alkylgruppen von 1 bis 3 C-Atomen, Chlor, Methoxi-, Ethoxi- oder Carbalkoxigruppen.

Zur Durchführung des erfindungsgemässen Verfahrens wird so vorgegangen, dass die Acyloxymethylenverbindung zusammen mit dem Alkohol ggf. in Gegenwart eines Katalysators bei der Reaktionstemperatur umgesetzt und nach Reaktionsende der Enoläther und/oder das Acetal durch übliche Aufarbeitungsmethoden wie Destillation oder Kristallisation isoliert wird.

Die Reaktanten können in beliebiger Reihenfolge zugegeben werden. Das Molverhältnis von Acyloxymethylenverbindung zu Alkohol beträgt im allgemeinen 1 zu 1 bis 1 zu 30, vorzugsweise 1 zu 2 bis 1 zu 15. Als Alkohole kommen insbesondere Alkylalkohole mit 1 bis 4 C-Atomen wie Methanol, Ethanol, Propanol oder Butanol in Frage, jedoch auch zweiwertige Alkohole $R^2(OH)_2$ wie z.B. Ethylenglykol oder Etheralkohole wie z.B. Methylglykol. Heteroatome in Diolen sind bevorzugt Stickstoff oder Sauerstoff, ggf. Schwefel.

Die Umsetzung kann in Abwesenheit oder bevorzugt in Gegenwart eines Katalysators erfolgen. Der Katalysator bewirkt im allgemeinen höhere Umsätze und Reaktionsgeschwindigkeiten. Als Katalysatoren kommen Protonen- oder Lewis-Säuren in Frage; als Beispiele sind zu nennen HCl, $H_2SO_4$, $KHSO_4$, $HNO_3$, $H_3PO_4$, $NH_4Cl$, p-Toluolsulfonsäure, $FeCl_3$, $AlCl_3$, $ZnCl_2$, $BF_3$, saure Kationenaustauscher, doch stellt diese Aufzählung keine Begrenzung dar. Der Katalysator wird in Mengen von 0,01 bis 40 Mol-%, vorzugsweise von 0,05 bis 20 Mol-% eingesetzt.

Die Reaktionstemperatur beträgt im allgemeinen 20 bis 250, vorzugsweise 50 bis 150 °C. Die Reaktion erfolgt im allgemeinen unter Rückflusssieden des Alkohols. Der Druck liegt bevorzugt bei Normaldruck, jedoch ist Überdruck bis 40 bar möglich. Die Reaktionszeit beträgt im allgemeinen 0,2 bis 5, bevorzugt 0,2 bis 3 Stunden.

Die Aufarbeitung erfolgt im allgemeinen so, dass ggf. nach Abtrennung oder Neutralisation des Katalysators bzw. Abtrennung seines Salzes der überschüssige Alkohol abdestilliert und der Enoläther und/oder das Acetal durch Destillation oder Kristallisation rein gewonnen wird. Die Neutralisation des Katalysators kann durch Basen wie z.B. Ammoniak, Alkalialkoholat, Alkali- oder Erdalkali-Hydroxide oder -Oxide erfolgen.

Die Umsetzung der Acyloxymethylenverbindung III mit Alkoholen ergibt in Abhängigkeit von der Art des Substituenten Y, von den Reaktionsbedingungen und den Aufarbeitungsbedingungen den Enoläther oder das Acetal oder Gemische hiervon.

Acyloxymethylenverbindungen der Formel III, in denen Y nur die Bedeutung CN oder $COOR^1$ hat, reagieren mit Alkoholen ausschliesslich zu den Enoläthern der Formel I.

Acyloxymethylenverbindungen III, in denen Y die Bedeutung von $R^3$ hat, reagieren in Abhängigkeit von den Reaktions- und Aufarbeitungsbedingungen zu den Enoläthern I oder den Acetalen IIa bzw. IIb oder deren Gemischen mit I. Dabei begünstigt ein geringer Alkoholüberschuss oder das Verbleiben des Katalysators im Reaktionsgemisch bei der Aufarbeitung und die Anwendung hoher Temperaturen bei der destillativen Aufarbeitung die Bildung des Enoläthers. Im Falle der Diole $R^2(OH)_2$ bilden sich Acetale der Formel IIb.

Die nach dem erfindungsgemässen Verfahren darstellbaren Enoläther und Acetale sind Zwi-

Beispiele

| Nr. | Acyloxymethylenverb. | Alkohol | Katalysator | Temp. (°C) | Zeit (h) | Aufarbeitung | Produkt | Ausb. % |
|---|---|---|---|---|---|---|---|---|
| 1. | 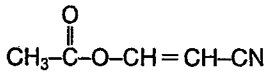 10,8 g 0,1 mol | $CH_3OH$ 32 g 1 mol | $AlCl_3$ 2,7 g 0,02 mol | 65 | 2 | mit $NaOCH_3$ pH 7, Salzabtrenng. | $(CH_3O)_2CH-CH_2-CN$ 9,0 g | 78,2 |
| 2. | 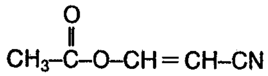 10,8 g 0,1 mol | $C_2H_5OH$ 46 g 1 mol | HCl 0,073 g 0,002 mol | 78 | 2 | – | $(C_2H_5O)_2CH-CH_2-CN$ 12,6 g | 88,0 |
| 3. | 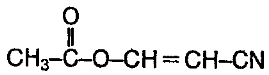 10,8 g 0,1 mol | 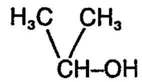 60 g 1 mol | HCl 0,2 g 0,005 mol | 82 | 3 | – | 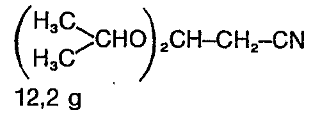 12,2 g | 71,3 |
| 4. | 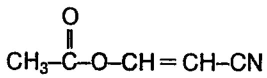 10,8 g 0,1 mol | $n-C_4H_9OH$ 74 g 1 mol | p-Toluolsulfon-säure 1,7 g 0,01 mol | 117 | 1 | mit Natriumbutylat pH 7, Salzabtrenng. | 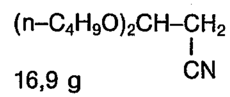 16,9 g | 84,8 |
| 5. | 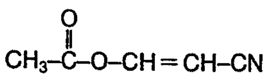 10,8 g 0,1 mol | 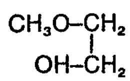 76 g 1 mol | $H_2SO_4$ 0,1 g 0,001 mol | 123 | 1 | – | $CH_3O-CH_2O-CH=CH-CN$ | 81,4 |
| 6. | 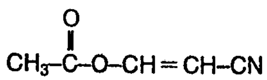 10,8 g 0,1 mol | 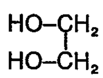 62,1 g 1 mol | $NH_4Cl$ 1,0 g 0,02 mol | 150 | 3 | – | 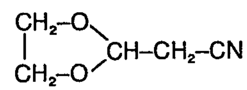 6,2 g | 54,9 |
| 7. | 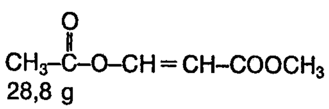 28,8 g 0,2 mol | $CH_3OH$ 48 g 1,5 mol | HCl 0,037 g 0,001 mol | 65 | – | mit $NaOCH_3$ pH 7, | $(CH_3O)_2CH-CH_2-COOCH_3$ 21,7 g | 73,3 |

Beispiele

| Nr. | Acyloxymethylenverb. | Alkohol | Katalysator | Temp. (°C) | Zeit (h) | Aufarbeitung | Produkt | Ausb. % |
|---|---|---|---|---|---|---|---|---|
| 8. | 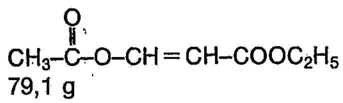 79,1 g 0,5 mol | $C_2H_5OH$ 230 g 5 mol | $KHSO_4$ 0,68 g 0,005 mol | 78 | 3 | mit $NaOC_2H_5$ pH 7 | 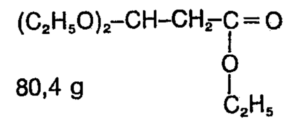 80,4 g | 84,5 |
| 9. | 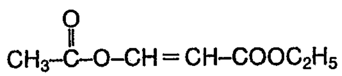 79,1 g 0,5 mol | $C_2H_5-OH$ 230 g 5 mol | $KHSO_4$ 0,68 g 0,005 mol | 78 | 3 | Abdestillieren v. Ethanol, 1 Std. 160°C (760 Torr) Vakuumdest. | $C_2H_5O-CH=CH-COOC_2H_5$ 49,0 g | 68,0 |
| 10. | 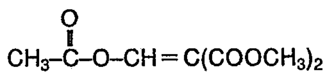 20,2 g 0,1 mol | $CH_3OH$ 32 g 1 mol | HCl 0,073 g 0,002 mol | 65 | 1 | – | $CH_3O-CH=C(COOCH_3)_2$ 13,9 g | 79,8 |
| 11. | 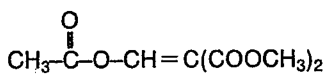 0,1 mol | $CH_3OH$ 32 g 1 mol | HCl 0,036 g 0,001 mol | 65 | 1 | – | $CH_3O-CH=C(COOCH_3)_2$ 15,0 g | 86,1 |
| 12. | 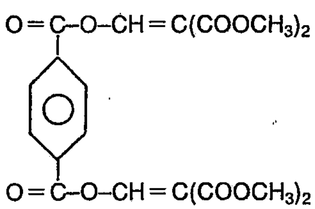 33,8 g 0,075 mol | $CH_3OH$ 48 g 1,5 mol | HCl 0,27 g 0,0075 mol | 65 | 2 | – | $CH_3O-CH=C(COOCH_3)_2$ 19,3 g | 73,9 |
| 13. | 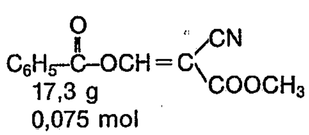 17,3 g 0,075 mol | $CH_3OH$ 32 g 1 mol | HCl 0,9 g 0,025 mol | 65 | 2 | – | 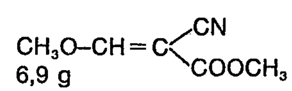 6,9 g | 65,2 |

| | | | | | | | | |
|---|---|---|---|---|---|---|---|---|
| 14. | 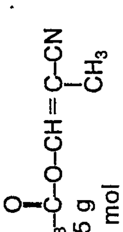 12,5 g 0,1 mol | $C_2H_5OH$ 55 g 1,2 mol | HCl 0,18 g 0,005 mol | 78 | 3 | – | 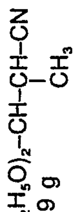 11,9 g | 75,8 |
| 15. | 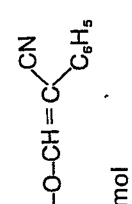 14,0 g 0,075 mol | $C_2H_5OH$ 46 g 1 mol | HCl 0,037 g 0,001 mol | 78 | 3 | – | 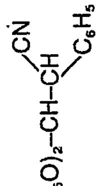 9,7 g | 58,9 |
| 16. | 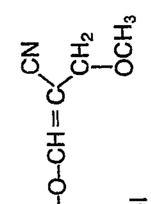 15,5 g 0,1 mol | $C_2H_5OH$ 46 g 1 mol | HCl 0,037 g 0,001 mol | 78 | 3 | – | 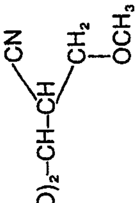 13,0 g | 69,4 |

schenprodukte z.B. zur Herstellung von pharmazeutischen Produkten.

Die Beispiele dienen der weiteren Erläuterung der Erfindung.

Die Beispiele sind tabellarisch aufgeführt.

Allgemeine Arbeitsvorschrift:

Der Alkohol, der Katalysator und als letzte Komponente die Acyloxymethylenverbindung werden zusammengegeben und bei der angegebenen Reaktionstemperatur zur Umsetzung gebracht. Ggf. nach Abtrennung oder Neutralisation des Katalysators bzw. Abtrennung seines Salzes wird der überschüssige Alkohol abdestilliert und schliesslich der Enoläther/das Acetal destillativ gereinigt. Die Produktcharakterisierung erfolgte durch Protonenresonanzanalyse, IR- und Passenspektroskopie.

## Patentansprüche

1. Verfahren zur Herstellung von Enoläthern der Formel

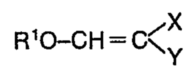 I

und/oder Acetalen der Formeln

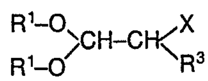 IIa, bzw.

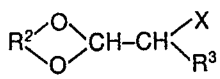 IIb,

worin $R^1$ geradkettige oder verzweigte Alkylreste mit 1 bis 20 C-Atomen oder $(CH_2)_m$-Cyc mit Cyc = isocyclische oder heterocyclische, einkernige oder mehrkernige aromatische oder cycloaliphatische Ringsysteme, welche gegebenenfalls Substituenten an den Ringen tragen, mit m = 0 bis 5, die Reste $(CH_2)_nOR^4$ oder $(CH_2-CH_2O)_nOR^4$ mit n = 1 bis 4 und mit $R^4$ = geradkettige oder verzweigte Alkylreste mit 1 bis 12 C-Atomen,

$R^2$ einen doppelt gebundenen Alkylen- oder Alkenylen-Rest mit 2 bis 6 C-Atomen, die ggf. durch ein oder mehrere Heteroatome unterbrochen sind,

$R^3$ = H, geradkettige oder verzweigte Alkylreste mit 1 bis 20 C-Atomen, geradkettige oder verzweigte Reste $(CH_2)_n$–CN, $(CH_2)_nCOOR^4$, $(CH_2)_nNR^5{}_2$, $(CH_2)_mOR^4$ oder $(CH_2)_m$ Cyc bedeuten, wobei n, m, $R^4$ und Cyc die genannte Bedeutung haben und $R^5$ H, geradkettige oder verzweigte Alkylreste mit 1 bis 12 C-Atomen, Cyc oder $COR^6$ und $R^6$ = H, geradkettige oder verzweigte Alkylreste mit 1 bis 12 C-Atomen oder Cyc ist, wobei die Reste $R^5$ gleich oder ungleich sein können;

X die Bedeutung CN oder $COOR^1$ hat und

Y die Bedeutung von $R^3$ oder von X hat,

dadurch gekennzeichnet, dass eine Acyloxymethylenverbindung der Formel

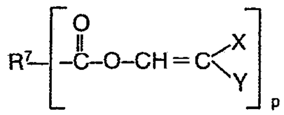

III,

worin X und Y die genannte Bedeutung haben und $R^7$ geradkettige oder verzweigte Alkyl-, Alkylen-, Alkenyl- oder Alkenylenreste mit 1 bis 12 C-Atomen, isocyclische oder heterocyclische Ringsysteme mit ein- oder mehrcyclischer Struktur oder $(CH_2)_n$ Cyc, wobei n und Cyc die oben genannte Bedeutung haben, bedeutet und p = 1 oder 2 ist, mit einem Alkohol der Formel

$R^1OH$ IV, oder $R^2(OH)_2$ V,

worin $R^1$ und $R^2$ die genannte Bedeutung haben, umgesetzt wird.

2. Verfahren nach Anspruch 1, dadurch gekennzeichnet, dass die Umsetzung bevorzugt in Gegenwart einer Protonen- oder Lewissäure erfolgt.

3. Verfahren nach den Ansprüchen 1 oder 2, dadurch gekennzeichnet, dass die Katalysatormenge 0,01 bis 40 Mol-%, vorzugsweise 0,05 bis 20 Mol-% beträgt.

4. Verfahren nach einem der Ansprüche 1 bis 3, dadurch gekennzeichnet, dass das Molverhältnis von Acyloxymethylenverbindung zu Alkohol 1 zu 1 bis 1 zu 30, vorzugsweise 1 zu 2 bis 1 zu 15, beträgt.

5. Verfahren nach einem der Ansprüche 1 bis 4, dadurch gekennzeichnet, dass die Reaktionstemperatur 20 bis 250 °C, vorzugsweise 50 bis 150 °C beträgt.

**Claims**

1. Process for the preparation of enol ethers of the formula

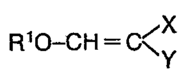

I

and/or acetals of the formula

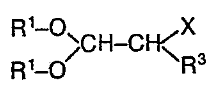

IIa, or

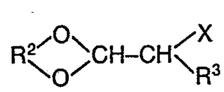

IIb,

wherein $R^1$ denotes straight-chained or branched alkyl residues with 1 to 20 C-atoms or $(CH_2)_m$-Cyc with Cyc = isocyclic or heterocyclic mononuclear or polynuclear aromatic or cycloaliphatic ring systems which optionally carry substituents on the ring, with m = 0 to 5, the residues $(CH_2)_nOR^4$ or $(CH_2CH_2O)_nOR^4$ with n = 1 to 4 and with $R^4$ = straight chained or branched alkyl residues with 1 to 12 C-atoms,

$R^2$ denotes a doubly bound alkylene or alkenylene residue with 2 to 6 C-atoms which are optionally interrupted by one or several hetero atoms,

$R^3$ = H, straight-chained or branched alkyl residues with 1 to 20 C-atoms, straight-chained or branched residues $(CH_2)_n$–CN, $(CH_2)_nCOOR^4$, $(CH_2)_nNR^5_2$, $(CH_2)_mOR^4$ or $(CH_2)_m$ Cyc, with n, m, $R^4$ and Cyc having the indicated meaning and $R^5$ is H, straight-chained or branched alkyl residues with 1 to 12 C-atoms, Cyc or $COR^6$ and $R^6$=H, straight-chained or branched alkyl residues with 1 to 12 C-atoms or Cyc, with the residues $R^5$ being able to be like or different,

X has the meaning CN or $COOR^1$ and

Y has the meaning of $R^3$ or of X,

characterised in that an acyloxymethylene compound of the formula

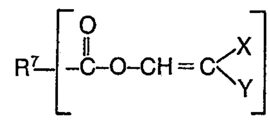

III,

wherein X and Y have the indicated meaning and $R^7$ means straight-chained or branched alkyl, alkylene, alkenyl or alkenylene residues with 1 to 12 C-atoms, isocyclic or heterocyclic ring systems with mono or polynuclear structure or $(CH_2)_n$ Cyc, with n and Cyc having the above-indicated meaning, and p = 1 or 2, is reacted with an alcohol of the formula

$R^1OH$ IV, or $R^2(OH)_2$ V,

wherein $R^1$ and $R^2$ have the indicated meaning.

2. Process according to claim 1, characterised in that the reaction takes place preferably in the presence of a proton or Lewis acid.

3. Process according to claims 1 or 2, characterised in that the catalyst quantity amounts to 0.01 to 40 mol%, preferably 0.05 to 20 mol%.

4. Process according to one of claims 1 to 3, characterised in that the molar ratio of acyloxymethylene compound to alcohol amounts to 1:1 to 1:30, preferably 1:2 to 1:15.

5. Process according to one of claims 1 to 4, characterised in that the reaction temperature amounts to 20 to 250 °C, preferably 50 to 150 °C.

**Revendications**

1. Procédé pour la préparation d'éthers énoliques de formule

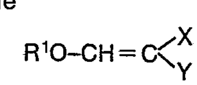

I

et/ou d'acétals de formules

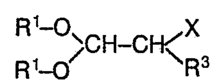

IIa, ou

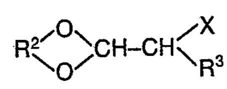

IIb,

où $R^1$ représente des radicaux alkyle à chaîne droite ou ramifiée ayant de 1 à 20 atomes de carbone, ou $(CH_2)_m$-Cyc, avec Cyc = des systèmes cycliques, isocycliques ou hétérocycliques,

aromatiques ou cycloaliphatiques, à un ou plusieurs noyaux, qui portent éventuellement des substituants sur les noyaux, avec m = 0 à 5, les radicaux $(CH_2)_nOR^4$ ou $(CH_2-CH_2O)_nOR^4$ avec n = 1 à 4 et avec $R^4$ = radicaux alkyle à chaîne droite ou ramifiée ayant de 1 à 12 atomes de carbone,

$R^2$ est un radical alkylène ou alcénylène doublement lié, à 2 à 6 atomes de carbone, et éventuellement interrompu par un ou plusieurs hétéroatomes,

$R^3$ = H, des radicaux alkyle à chaîne droite ou ramifiée ayant de 1 à 20 atomes de carbone, des radicaux à chaîne droite ou ramifiée $(CH_2)_n-CN$, $(CH_2)_nCOOR^4$, $(CH_2)_nNR^5_2$, $(CH_2)_mOR^4$ ou $(CH_2)_mCyc$, où n, m, $R^4$ et Cyc ont les significations déjà données et $R^5$ est H, ou représente des radicaux alkyle à chaîne droite ou ramifiée ayant de 1 à 12 atomes de carbone, Cyc ou $COR^6$, et $R^6$ = H, ou représente des radicaux alkyle à chaîne droite ou ramifiée ayant de 1 à 12 atomes de carbone, ou Cyc, les radicaux $R^5$ pouvant être identiques ou différents,

X désigne CN ou $COOR^1$ et

Y désigne $R^3$ ou X,

caractérisé en ce qu'on fait réagir un composé acyloxyméthylène de formule

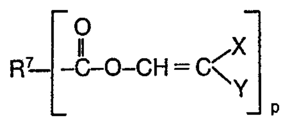

III,

dans laquelle X et Y ont les significations données ci-dessus et $R^7$ signifie un radical alkyle, alkylène, alcényle ou alcénylène à chaîne droite ou ramifiée ayant de 1 à 12 atomes de carbone, des systèmes cycliques isocycliques ou hétérocycliques à structure monocyclique ou polycyclique ou $(CH_2)_nCyc$, où n et Cyc ont les significations données ci-dessus, et p = 1 ou 2, sur un alcool de formule

$$R^1OH \quad IV \quad ou \quad R^2(OH)_2 \quad V,$$

où $R^1$ et $R^2$ ont les significations données ci-dessus.

2. Procédé selon la revendication 1, caractérisé en ce que la réaction est de préférence mise en œuvre en présence d'un acide protonique ou d'un acide de Lewis.

3. Procédé selon les revendications 1 ou 2, caractérisé en ce que la quantité de catalyseur est de 0,01 à 40% en moles, de préférence de 0,05 à 20% en moles.

4. Procédé selon l'une des revendications 1 à 3, caractérisé en ce que le rapport molaire entre le composé acyloxyméthylène et l'alcool est de 1:1 à 1:30, de préférence de 1:2 à 1:15.

5. Procédé selon l'une des revendications 1 à 4, caractérisé en ce que la température de réaction est de 20 à 250 °C, de préférence de 50 à 150 °C.